(19) Europäisches Patentamt
European Patent Office
Office européen des brevets

(11) **EP 2 344 033 B1**

(12) **EUROPEAN PATENT SPECIFICATION**

(45) Date of publication and mention
of the grant of the patent:
**05.06.2013 Bulletin 2013/23**

(51) Int Cl.:
**A61B 5/02** (2006.01)     **A61B 5/026** (2006.01)
**A61B 5/053** (2006.01)

(21) Application number: **09748147.7**

(22) Date of filing: **07.10.2009**

(86) International application number:
**PCT/IB2009/054394**

(87) International publication number:
**WO 2010/041206 (15.04.2010 Gazette 2010/15)**

(54) **DIAGNOSIS OF ACUTE STROKES**

DIAGNOSE VON AKUTEN SCHLAGANFÄLLEN

DIAGNOSTIC D'ACCIDENTS CÉRÉBROVASCULAIRES AIGUS

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **07.10.2008 US 103287 P**

(43) Date of publication of application:
**20.07.2011 Bulletin 2011/29**

(73) Proprietor: **Orsan Medical Technologies Ltd.
42504 Natania (IL)**

(72) Inventors:
• **POUPKO, Ben, Zion
74051 Nes Ziona (IL)**
• **RAPPAPORT, Alon
69407 Tel-Aviv (IL)**
• **BEN-ARI, Shlomi
30550 Binyamina (IL)**

(74) Representative: **Dennemeyer & Associates S.A.
55, rue des Bruyères
1274 Howald (LU)**

(56) References cited:
**US-A1- 2007 287 899     US-A1- 2008 004 904**

• E. G. HOEFFNER ET AL: 'Cerebral Perfusion CT:
Technique and Clinical Applications'
RADIOLOGY vol. 231, no. 3, 01 June 2004, pages
632 - 644, XP055028383 DOI: 10.1148/radiol.
2313021488 ISSN: 0033-8419

## Description

### FIELD AND BACKGROUND OF THE INVENTION

The present invention, in some embodiments thereof, relates to a system for diagnosing and/or choosing therapy for acute strokes using impedance plethysmography (IPG) and/or photoplethysmography (PPG) and, more particularly, but not exclusively, to a method and system for choosing or rejecting thrombolytic therapy for acute strokes.

A number of cerebral hemodynamic parameters may be clinically useful for diagnosing strokes, trauma, and other conditions that can affect the functioning of the cerebrovascular system. These parameters include regional cerebral blood volume, cerebral blood flow, cerebral perfusion pressure, mean transit time, time to peak, and intracranial pressure. Many methods that are used to measure these parameters, while giving accurate results, are not practical to use for continuous monitoring, or for initial diagnosis outside a hospital setting, because they are invasive, or because they require expensive and/or non-portable equipment. Such methods include inserting a probe into the cerebrospinal fluid or into an artery, computed tomography (CT), perfusion computed tomography (PCT), positron emission tomography (PET), magnetic resonance imaging (MRI), and transcranial Doppler ultrasound (TCD). Some of this prior art is reviewed in US patent application 11/610,553, published as US2007/0287899 and WO2008/072223, which is regarded as the closest prior art to the present application, and in the patent application publications WO 03/059164, US 2005/0054939, WO 2006/006143, WO 2006/011128, WO 2006/134501, and WO 2008/07223.

The use of perfusion computed tomography for finding cerebral hemodynamic parameters, and the use of these parameters in evaluating and choosing courses of treatment for stroke patients, is described by Christian Baumgartner et al, "Functional Cluster Analysis of CT Perfusion Maps: A New Tool for Diagnosis of Acute Strokes," J. of Digital Imaging 18, 219-226 (2005); by Roland Bruening, Axel Kuettner and Thomas Flohr, Protocols for Multislice CT (Springer, 2005), especially on page 96; by Ellen G. Hoeffner et al, "Cerebral Perfusion CT: Technique and Clinical Applications," Radiology 231, 632-644 (2004); and by Hiroshi Hagiwara et al, "Predicting the Fate of Acute Ischemic Lesions Using Perfusion Computed Tomography," J. Comput. Assist. Tomogr. 32, 645-650 (2008).

A. M. Weindling, N. Murdoch, and P. Rolfe, "Effect of electrode size on the contributions of intracranial and extracranial blood flow to the cerebral electrical impedance plethysmogram," Med. & Biol. Eng. & Comput. 20, 545-549 (1982) describes measurements of blood flow in the head, using separate current and voltage electrodes on the front and back of the head, and measuring the peak-to-peak change in impedance over a cardiac cycle to find the blood flow. A tourniquet was placed around the head to temporarily stop the scalp blood flow, and then released, in order to determine how much of the measured blood flow was due to scalp blood flow, and how much was due to intracranial blood flow. The scalp blood flow was considered to be completely cut off when there was no detectable variation in the signal from a PPG sensor at the cardiac frequency.

J. Gronlund, J. Jalonen, and I. Valimaki, "Transcephalic electrical impedance provides a means for quantifying pulsatile cerebral blood volume changes following head-up tilt," Early Human Development 47 (1997) 11-18, describe electrical impedance measurements of the head in premature newborn infants. Changes in impedance associated with the cardiac cycle are said to reflect changes in total cerebral blood volume, and earlier papers are referenced which are said to demonstrate this. Variability in impedance, in the range of 1.5 to 4 Hz, was found to decrease by 27%, on average, when the infants' heads were tilted up by 20 degrees. An earlier paper describing related research by the same group is J. Gronlund et al, "High Frequency Variability of Transcephalic Electrical Impedance: A New Parameter for Monitoring of Neonatal Cerebral Circulation?", Proceedings of the Annual International Conference of the Engineering in Medicine and Biology Society, Paris, October 29-November 1, 1992, New York, IEEE, US, Vol. 6 Conf. 14, 29 October 1992, pages 2513-2515.

Rheoencephalography (REG) is a technique that uses bio-impedance measurements of the head to obtain information on about cerebral blood circulation and circulatory problems. Generally, changes in impedance Z across the head, for a particular arrangement of electrodes, are measured as a function of time t over a cardiac cycle, and sometimes over a breathing cycle, due to changes in the volume and distribution of blood in the head. As described by W. Traczewski et al, "The Role of Computerized Rheoencephalography in the Assessment of Normal Pressure Hydrocephalus," J. Neurotrauma 22, 836-843 (2005), REG is commonly used to measure or diagnose problems with circulatory resistance, and problems with arterial elasticity. In patients with normal pressure hydrocephalus, for example, Traczewski et al find two different patterns in Z(t), depending on the elasticity of the small cerebral arteries. The pattern of Z(t) seen in a given patient is said to be useful for making predictions about the likely outcome of different treatments for the hydrocephalus. These patients all had similar, normal values of ICP.

G. Bonmassar and S. Iwaki, "The Shape of Electrical Impedance Spectrosopy (EIS) is altered in Stroke Patients," Proceedings of the 26th Annual Conference of IEEE EMBS, San Francisco, CA, USA, September 1-5, 2004, describes a system that uses electrical impedance to measure an asymmetry in the distribution of cerebral spinal fluid that is present in stroke patients, but not in healthy volunteers. The system uses 10 electrodes placed symmetrically around the subject's head, and passes white noise current at 0 to 25 kHz between any selected pair of electrodes, while measuring the potentials at all the electrodes. The system was found to work best if current was passed between the front and back of the head; but the paper also describes passing current between symmetrically placed electrodes on the left and right sides of the head.

WO 02/071923 to Bridger et al describes measuring and analyzing pulse waveforms in the head obtained from acoustic signals. Head trauma patients, and to a lesser extent stroke patients, are found to have differences from normal subjects. Trauma and stroke patients are found to have higher amplitudes at harmonics of the heart rate, at 5 to 10 Hz, than normal subjects do.

Yu. E. Moskalenko et al, "Slow Rhythmic Oscillations within the Human Cranium: Phenomenology, Origin, and Informational Significance," Human Physiology 27, 171-178 (2001), describes the use of electrical impedance measurements of the head, and TCD ultrasound measurements, to study slow waves, at frequencies of 0.08 to 0.2 Hz, that are apparently related to regulation of blood supply and oxygen consumption in the brain, and the circulation of cerebrospinal fluid. The studies were done with healthy subjects and with patients suffering from intracranial hypertension. A. Ragauskas et al, "Implementation of non-invasive brain physiological monitoring concepts," Medical Engineering and Physics 25, 667-687 (2003), describe the use of ultrasound to non-invasively monitor such slow waves, as well as pulse waves at the cardiac frequency, in intracranial blood volume, in head injury patients, and find that they can be used to determine intracranial pressure.

Additional background art includes WO 02/087410 to Naisberg et al; Kidwell CS et al, Comparison of MRI and CT for detection of acute intracerebral hemorrhage. JAMA; 2004: 292: 1823-1830; Horowitz SH et al, Computed tomographic-angiographic findings within the first 5 hours of cerebral infarction, Stroke; 1991: 22 1245-1253; The ATLANTIS, ECASS, and NINDS rt-PA study group investigators, Association of outcome with early stroke treatment: Pooled analysis of ATLANTIS, ECASS, and NINDS rt-PA stroke trials, Lancet; 363: 768-774; Albers G et al, Antithrombotic and thrombolytic therapy for ischemic stroke: The seventh ACCP conference on antithrombotic and thrombolytic therapy, Chest 2004; 126: 483-512; Kohrmann M et a,. MRI versus CT-based thrombolysis treatment within and beyond the 3 hour time window after stroke onset: a cohort study, Lancet Neurol 2006; 5:661-667; Albers GW et al, Magnetic resonance imaging profiles predict clinical response to early reperfusion: The diffusion and perfusion imaging evaluation for understanding stroke evolution (DEFUSE) study, Ann Neurol 2006; 60: 508-517; Johnston SC et al, National stroke association guidelines for the management of transient ischemic attacks, Ann Neurol 2006; 60: 301-313.

SUMMARY OF THE INVENTION

An aspect of some embodiments of the invention concerns evaluating acute stroke patients using IPG and/or PPG, for example to determine which ones are likely to benefit from thrombolytic therapy.

There is thus provided, in accordance with an exemplary embodiment of the invention, a system as defined in claim 1.

Further optional features are defined in dependent claims 2-18.

Unless otherwise defined, all technical and/or scientific terms used herein have the same meaning as commonly understood by one of ordinary skill in the art to which the invention pertains. Although materials similar or equivalent to those described herein can be used in the practice or testing of embodiments of the invention, exemplary materials are described below. In case of conflict, the patent specification, including definitions, will control. In addition, the materials, methods, and examples are illustrative only and are not intended to be necessarily limiting.

Implementation of the system of embodiments of the invention can involve performing or completing selected tasks manually, automatically, or a combination thereof. Moreover, according to actual instrumentation and equipment of embodiments of the system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 schematically shows a cerebral perfusion diagnosing system, diagnosing an acute stroke patient, according to an exemplary embodiment of the invention ;
FIG. 2 is a flowchart showing a method of diagnosing a patient using the system in FIG. 1;
FIG. 3 is a more detailed view schematically showing an exemplary IPG electrode structure and PPG sensor that can be used in the system in FIG. 1, placed on the head of a patient;
FIG. 4 is a more detailed schematic view of the IPG electrode structure shown in FIG. 3;
FIG. 5 is a more detailed schematic view of the PPG sensor shown in FIG. 3; and
FIG. 6A schematically shows IPG and PPG signals for a patient with high global CBV, and FIG. 6B schematically shows IPG and PPG signals for a patient with low global CBV, illustrating a method of analyzing IPG and PPG signals according to an exemplary embodiment of the invention.

DESCRIPTION OF EMBODIMENTS OF THE INVENTION

The present invention, in some embodiments thereof; relates to a system for diagnosing and/or choosing therapy for acute strokes using impedance plethysmography (IPG) and/or photoplethysmography (PPG) and, more particularly, but not exclusively, to a method and system for choosing or rejecting thrombolytic therapy for acute strokes.

An example illustrating the invention concerns a method of evaluating a patient suspected of suffering from an acute stroke, and in particular determining whether the patient is likely to benefit from thrombolytic therapy. The method uses IPG and/or PPG signals to obtain measures of cerebral hemodynamics in the patient, for example estimates of standard cerebral hemodynamic parameters such as cerebral blood flow (CBF), cerebral blood volume (CBV), mean transit time (MTT), and time to peak (TTP). These measures may be used to determine whether the patient has suffered from an ischemic stroke, a hemorrhagic stroke, or another medical condition entirely, such as brain tumor which is producing clinical symptoms similar to a stroke. If the patient has suffered an ischemic stroke, these measures may be used to estimate the size of the core and penumbra of the ischemia. Generally, only in patients with intermediate sized ischemic strokes, where there is a relatively large penumbra of brain tissue that can potentially be saved, are the benefits of thrombolytic therapy likely to outweigh the risks. Using IPG and/or PPG signals to evaluate the patient is potentially advantageous, because prior art methods of determining cerebral hemodynamic parameters, such as perfusion CT and MRI, are expensive and are available only in certain hospitals. IPG and PPG signals, on the other hand, can be measured by an emergency medical responder, at a patient's home or in an ambulance, or promptly in any hospital emergency room, allowing thrombolytic therapy, if appropriate, to be started quickly, within the time window when it is likely to help. IPG and/or PPG signals can also be used to evaluate ischemic stroke patients who are past the normal time limit for thrombolytic therapy, but whose cerebral hemodynamic parameters indicate they would still be likely benefit from it.

Typically, CBV is depressed only in the core region of an ischemia, where blood flow has stopped completely and the tissue is likely to progress to infarction, even if blood flow can be restored. On the other hand, CBF is typically depressed, and TTP is typically longer than normal, throughout the penumbra, where brain tissue is in danger but can potentially be saved. Optionally, the IPG and/or PPG signals are used to estimate CBV, to determine the size of the ischemic core region, and CBF or TTP or both, to determine the size of the penumbra. Generally, thrombolytic therapy is likely to benefit patients with a relatively large penumbra outside the ischemic core region.

A variety of methods of analyzing IPG and PPG signals are optionally used to estimate standard parameters or to obtain other measures of cerebral hemodynamics. Typically the measures depend on the behavior of the signal as a function of phase of the cardiac cycle, although measures based on behavior over longer time scales, such as a slow wave amplitude, may also be found. The signals may be smoothed, or averaged over multiple cardiac cycles, or transformed in other ways, and noisy or outlying cardiac cycles may be excluded. The measures may pertain to an effective rise time interval of the signal during a cardiac cycle, defined in various ways, or to an effective fall time interval. Obtaining the measures may involve comparing measures found from substantially the same algorithm applied to different signals, for example comparing IPG and PPG signals, or compaiing signals based on data pertaining to different sides of the head, or comparing a signal from data pertaining symmetrically to both sides of the head to a signal from data pertaining to only one side of the head. The measures may be dimensionless, not depending on an amplification gain of the signals. Optionally, ECG data is used in obtaining the measures, for example ECG data is used to calibrate the timing of a feature of the signal relative to the cardiac cycle.

Before explaining at least one embodiment of the invention in detail, it is to be understood that the invention is not necessarily limited in its application to the details of construction and the arrangement of the components set forth in the following description and/or illustrated in the drawings and/or the Examples. The invention is capable of other embodiments or of being practiced or carried out in various ways.

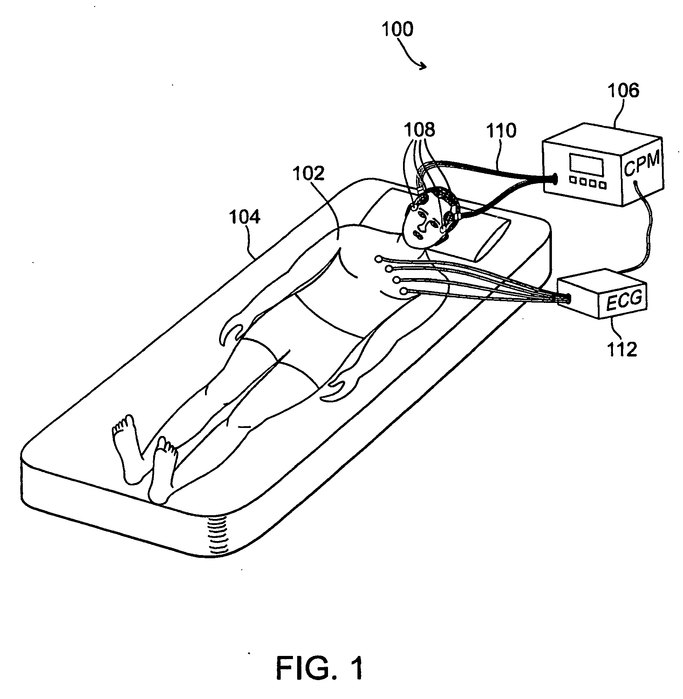

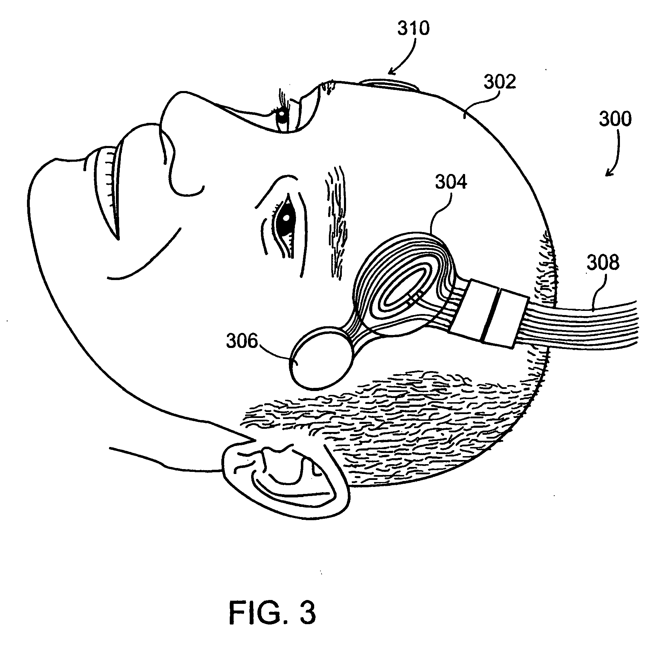

Referring now to the drawings, FIG. 1 illustrates a cerebral perfusion monitoring system 100, being used to evaluate a patient 102 suspected of having suffered an acute stroke. Optionally, system 100 is operated by emergency medical personnel or other trained first responders, and patient 102 may be lying on a gurney 104 in an ambulance or hospital emergency room, or even at home while awaiting an ambulance. A controller 106 is connected to sensors 108, placed on the patient's head, by cables 110. The sensors include electrodes for IPG, and/or PPG sensors, which generate IPG and PPG signals analyzed by controller 106. The sensors are shown in more detail below, in FIGS. 3-5. Optionally, controller 106 includes a display which shows estimated values for cerebral hemodynamic parameters, calculated by controller 106 from the IPG and PPG signals. Optionally, an ECG device 112 is connected to ECG electrodes placed on the patient's chest, and ECG data is used by controller 106 in analyzing the signals from sensors 108.

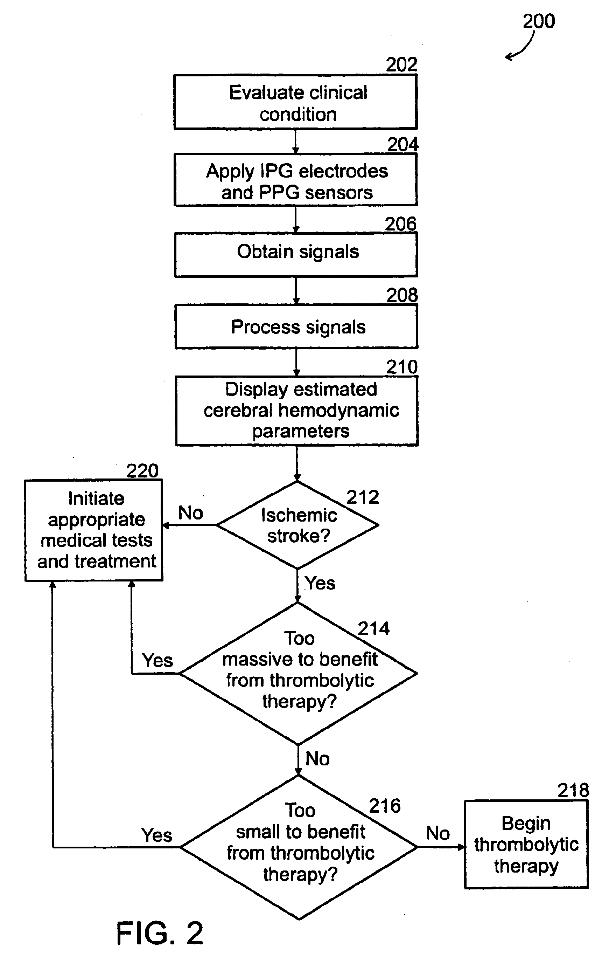

FIG. 2 shows a flowchart 200 for a method of evaluating a patient suspected of suffering from a stroke, using system 100 in FIG. 1. At 202, the clinical condition of the patient is evaluated. Clinical symptoms are generally used together with an analysis of IPG and PPG signals in evaluating the patient, and the procedure used in obtaining and processing IPG and PPG signals may depend on the clinical symptoms. For example, some methods of analyzing the signals make a distinction between signals obtained from the same side of the head that the stroke occurred on, and signals obtained from the opposite side of the head. Clinical symptoms, such as hemiplegia, may provide an indication of which side of the head the stroke occurred on.

At 204, IPG electrodes and/or PPG sensors are placed on the patient's head. In some embodiments of the invention, electrodes and/or PPG sensors may also be placed on the patient's neck, for example to measure a signal of blood flow in the carotid artery or another artery in the neck. Details of how electrodes and sensors are placed on the patient's head are described below in connection with FIG. 3.

At 206, one or more IPG signals from the IPG electrodes, and/or one or more PPG signals from the PPG sensors, are obtained by controller 106, and are processed by the controller at 208. Examples of how this may be done are given below, under the heading "Exemplary methods of analyzing IPG and PPG signals." Controller 106 calculates from the signals one or more estimated cerebral hemodynamic parameters, such as CBF, CBV, MTT, and TTP, or other measures of cerebral hemodynamics, which can be used to help diagnose an acute stroke and find a course of therapy for it, in particular to decide whether or not the patient would be likely to benefit from thrombolytic therapy. The inventors have found, in clinical tests, that estimates of regional, hemispheric and global cerebral hemodynamic parameters, calculated from IPG and PPG signals, have correlations of about 0.5 to 0.7 with the same cerebral hemodynamic parameters measured by perfusion CT, across a sample of many different patients, with the parameters varying over a range of a factor of 2 or 3.

The estimated cerebral hemodynamic parameters or other measures are optionally displayed at 210. At 212, a rule is applied, by medical personnel using the displayed values, and/or by software run by the controller, matching the measures to a particular disease, such as an ischemic stroke or another medical condition, and/or to a a particular choice of therapy. If the patient did suffer an ischemic stroke, regional or hemispheric CBF would be expected to be substantially below normal values on the side of the head where the stroke occurred, for example below a threshold that is between 20 and 50 milliliters per 100 grams per minute, and to be substantially below global CBF, or below regional or hemispheric CBF on the other side of the head, for example by at least 20%, or at least 30%, or at least 50%. It should be noted that these thresholds, and the other thresholds described for 212, 214 and 216, need not be constant for all patients, but may depend on the values of other parameters, or on the patient's body temperature and metabolism, or on other factors. Additionally or alternatively, TTP may be used to evaluate whether the patient suffered an ischemic stroke, with TTP expected to be higher than normal on the side of the head where an ischemic stroke occurred, for example higher than a threshold that is between 30 and 40 seconds, and higher than on the other side of the head, for example by at least 10%, or at least 20%, or at least 50%. On the other hand, a hemorrhagic stroke may lead to depressed global CBF, and increased global MTT and TTP, as intracranial pressure builds up, without a large difference between the hemispheres. And other conditions that can cause symptoms similar to stroke, such as a brain tumor, might be expected not to cause a decrease in CBF at all, and possibly to cause higher than normal values of CBF on one or both sides of the head, due to angiogenesis.

If the estimated parameters indicate that the patient did suffer an ischemic stroke, then an evaluation is made at 214, by medical personnel and/or by software, as to whether the stroke is likely to be too massive for the patient to benefit from thrombolytic therapy. In such a massive stroke, the core region of the ischemia, where tissue is likely to progress to infarction even if blood flow is restored, may be relatively large, and there may be relatively little penumbra, where brain tissue can potentially be saved by restoring blood flow. In such a patient, there may be little potential benefit from thrombolytic therapy, and a large increased risk of hemorrhagic transformation of the ischemia if thrombolytic therapy is used. A massive ischemia of this sort may be indicated by regional or hemispheric CBV that is much lower than normal on the side of the head where the stroke occurred, for example lower than a threshold that is between 3 and 4 milliliters per 100 grams, and lower than global CBV, and regional and hemispheric CBV on the other side of the head, for example by at least 20%, or at least 30%, or at least 50%. Optionally, a patient is considered a good candidate for thrombolytic therapy only if at least one of regional and hemispheric CBV is above these thresholds. This is because CBV tends to be depressed only in the core region of the ischemia, but not in the penumbra where cerebral compensatory mechanisms can still operate to increase CBV in response to the ischemia.

If the estimated parameters indicate that the ischemia is not too massive, then an evaluation is made at 216, by medical personnel and/or by software, as to whether the ischemia is likely to be too small for the patient to benefit from thrombolytic therapy. A patient with a sufficiently small ischemia is not likely to benefit from thrombolytic therapy, since such a patient is likely to fully recover brain function in the penumbral tissue even without thrombolytic therapy, but thrombolytic therapy carries an increased risk of hemorrhagic transformation of the ischemia, or cerebral hemorrhage elsewhere. Such a condition may be indicated by relatively small decreases in regional and hemispheric CBF and CBV on the side of the stroke, and relatively small increases in TTP on the side of the stroke. For example, such a condition may be indicated by regional CBV greater than a threshold that is between 1.5 and 2.5 milliliters per 100 grams, and less than global or hemispheric CBV by no more than 70% or 50% or 25%. Baumgartner et al, cited above, found that TTP is more sensitive than CBF to reductions in blood flow due to an ischemic stroke, so the change in TTP may be an especially sensitive way to evaluate whether the ischemia is small enough so that brain function is likely to recover without thrombolytic therapy.

If the parameters indicate that the patient is likely to benefit from thrombolytic therapy, then thrombolytic therapy is begun, at 218. Optionally, the patient is monitored after receiving thrombolytic therapy, by a system similar to system 100, as described in the co-filed patent application titled "Monitoring of Acute Stroke Patients." If at any stage in the decision process, it is decided that the patient is not likely to benefit from thrombolytic therapy, then appropriate tests are done, and/or appropriate treatment is initiated, at 220.

FIG. 3 shows a combination sensor 300 for a cerebral perfusion monitor system, in place on the head of a patient 302. Another combination sensor 310, optionally a mirror image of sensor 300, is optionally used on the other side the patient's head, and is mostly hidden in FIG. 3. This sensor design is optionally used for sensors 108 in FIG. 1. Sensor 300 comprises an IPG electrode structure 304, optionally elliptical in shape, and optionally placed at a corner of the patient's forehead, optionally with an electrically conductive gel to assure good electrical contact with the skin. A PPG sensor 306, optionally circular, is optionally placed on the patient's temple. A cable 308 connects sensor 300 to the controller of the cerebral fusion monitor, for example controller 106 in FIG. 1. The cable optionally contains eight wires, including two wires used for electrode 304, and four wires used for PPG sensor 306 (two wires each for a light source and a light detector). Two of the wires in the cable are not used in sensor 300, but are included for use in a new design, under development, that will use two IPG electrodes on each side of the head.

Alternatively, any other design of IPG electrodes and/or PPG sensors, combined in one structure or separate, may be used, including any prior art design or off-the-shelf design for IPG electrodes and/or PPG sensors. The system need not use both IPG electrodes and PPG sensors, but optionally only uses one or the other.

The combination sensors used on the two sides of the patient's head are optionally placed at positions and orientations that are mirror images of each other, or nearly mirror images of each other, with respect to the bilateral symmetry plane of the head. Similarly, the two combination sensors are constructed to be mirror images of each other, or nearly mirror images of each other. Using sensors with such symmetry in design and location has the potential advantage that, by comparing measurements that are substantially mirror images of each other, they can be used to detect even small asymmetries in blood circulation in the head, which could be indicative of a stroke. In cases where the electrode and sensor configurations are said to be "nearly mirror images," the corresponding electrodes and sensors on the two sides of the head are all placed at locations that are mirror images of each other, to within 2 cm, or 1 cm, or 5 mm, or 2 mm, or 1 mm, or to within whatever precision the head is bilaterally symmetric. Alternatively, the corresponding electrodes and sensors are close enough to being placed in mirror image positions, that any differences in left and right hemisphere cerebral hemodynamic parameters inferred from the IPG and PPG signals from those misplaced sensors and electrodes will be small, by at least a factor of 2, or 5, or 10, or 20, compared to real differences in left and right hemisphere cerebral hemodynamic parameters typically found in ischemic stroke patients, or compared to the ranges in the values of these parameters typically seen among a random sample of ischemic stroke patients. Two measurements are "substantially mirror images of each other" if they are made with corresponding sensors and/or electrodes that are nearly mirror images in their configuration. Two measurements that are mirror images of each other, but are not identical, because each of the measurements is asymmetrical with respect to the bilateral symmetry of the head, should produce identical signals if the blood circulation in the head is bilaterally symmetric, as it normally is in a healthy subject. Any differences in such pairs of signals can reveal asymmetries in blood circulation in the head.

In some patients, previous trauma to the scalp or the brain, or previous brain surgery, may cause large asymmetries in the impedance of the head, so that asymmetry in cerebral blood circulation cannot be inferred simply from differences in the impedance signals from two mirror image measurements. Similarly, massive and asymmetric scarring from a burn or other trauma may cause asymmetries in PPG signals from symmetrically placed sensors on opposite sides of the head. Even in these patients, it might be possible to detect changes in the asymmetry of cerebral blood circulation, from changes in a difference between mirror image IPG or PPG signals, if the initial differences are properly calibrated.

In some embodiments of the invention, additional electrodes and/or PPG sensors are used. For example, there may be two electrodes on each side of the head, allowing impedance measurements to be made asymmetrically, for example locally on each side of the head. A number of such options are described in the co-filed application titled "Measurement of Cerebral Hemodynamic Parameters," cited above. As used herein, an impedance measurement is called "asymmetric" if it is neither symmetric (such as current going from the middle of the forehead to the back of the head) or antisymmetric (such as current going from the right temple to the left temple).

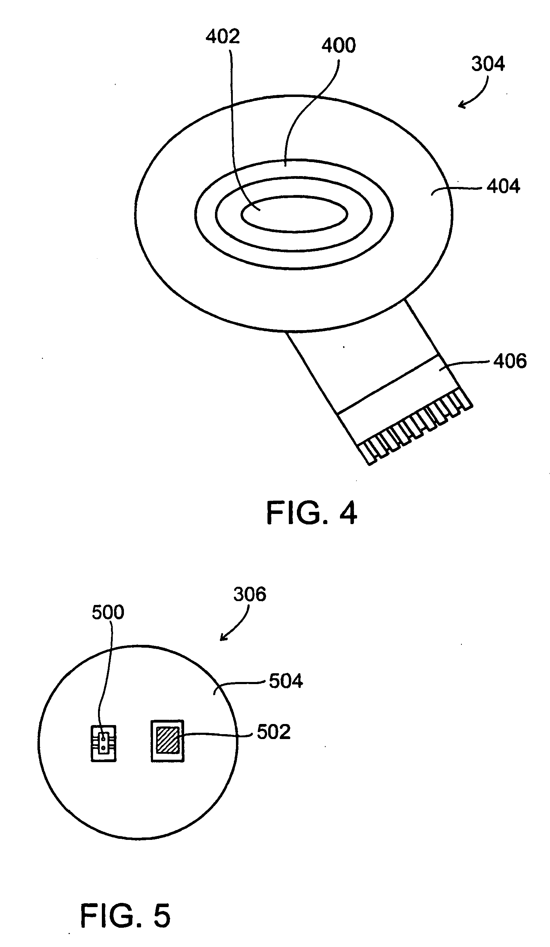

FIG. 4 shows electrode structure 304 in more detail. An elliptical ring-shaped current electrode 400 surrounds an elliptical voltage electrode 402. One of the wires in cable 308 connects to the current electrode, which passes current through the head, and one of the wires connects to the voltage electrode, which measures electric potential through a high impedance circuit, and passes very little current. Both are imbedded in an insulating holder 404, and a connector 406 snaps into a connector on the end of cable 308, shown in FIG. 3. Some of the potential advantages of using a ring-shaped current electrode surrounding a central voltage electrode are described in two related patent applications cited above, US patent application 10/893,570, published as US2005/0054939, and PCT application PCT/IL2005/000632, published as WO2006/011128, although in those applications the electrodes are circular rather than elliptical. The ring-shaped current electrode may produce a broader distribution of current, resulting in more current going through the brain and less current going through the scalp, than if a more compact current electrode of the same area were used. The separate high-impedance voltage electrode, insulated from the current electrode, may effectively measure the voltage drop across the interior of the skull, with relatively little less contribution from the high impedance skin and skull, than if the same electrode were used for passing current and measuring voltage. For safety reasons, the electrodes use a frequency of at least a few kHz, and currents no greater than 2.5 mA. For the test data shown below in the Examples, a frequency of 25 kHz and current of 1 mA or less was used.

FIG. 5 shows a more detailed view PPG sensor 306, showing the surface of the sensor that is in contact with the skin. The sensor comprises a red LED 500, and a photodiode 502, imbedded in an opaque holder 504 that keeps out stray light. A suitable LED is, for example, model TF281-200, sold by III-V Compounds. A suitable photodiode is, for example, model TFMD5000R, also sold by III-V Compounds. Red light from the LED scatters from blood in the skin, with relatively little absorption compared to blue or green light. The amplitude of scattered light detected by the photodiode, which is optionally further shielded from stray light by a red filter that covers it, increases with increasing blood volume in the skin in the immediate vicinity of the LED and photodiode, and exhibits a characteristic rising and falling pattern over a cardiac cycle.

Exemplary methods of analyzing IPG and PPG signals

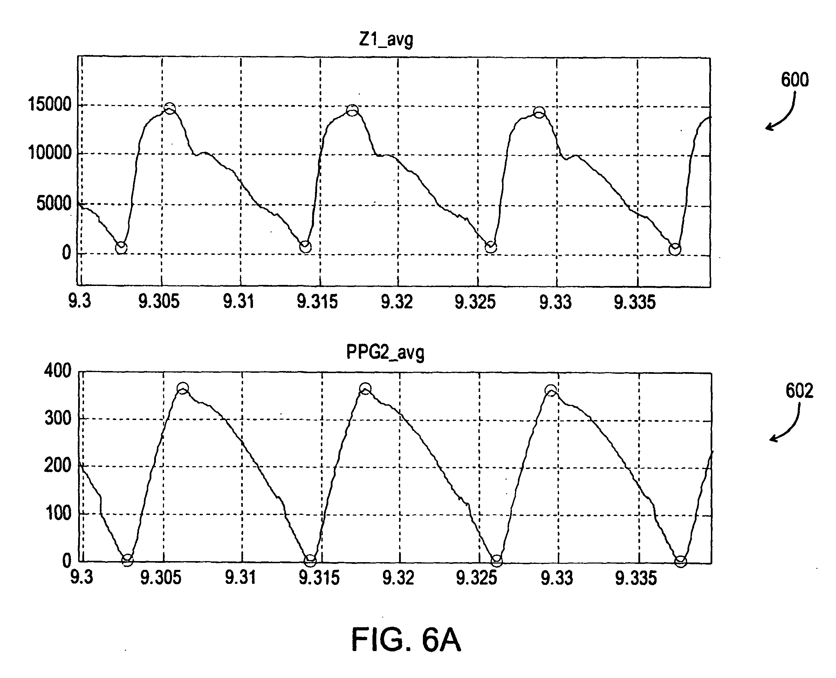

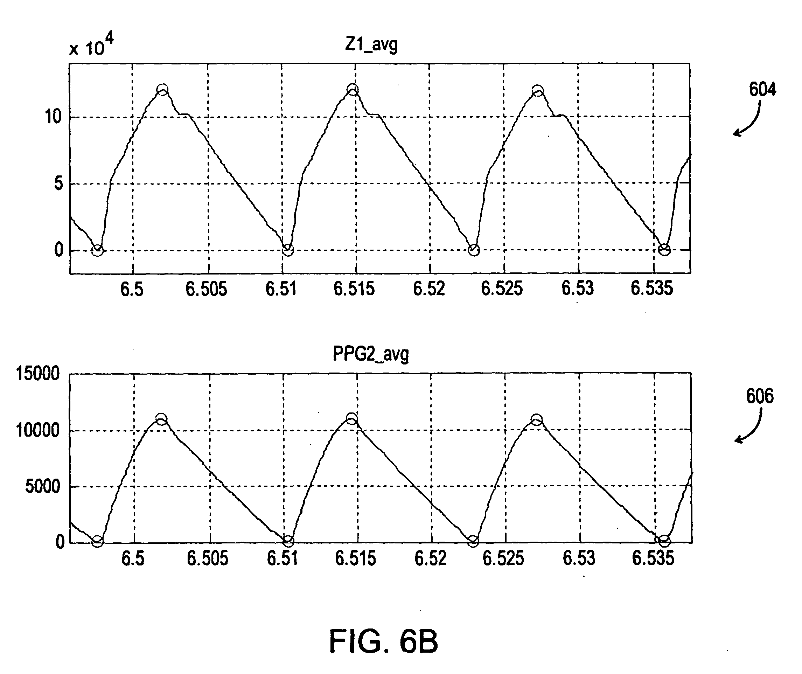

A number of methods of analyzing IPG and PPG signals have been found by the inventors to be useful for estimating standard cerebral hemodynamic parameters, as shown by results of a clinical study described below in the Examples. Most of these methods involve analysis of features of the signal that approximately repeat each cardiac cycle. For those features, noise can optionally be reduced by detrending the signal, so that it is always at the same level at the diastolic point of each cycle, by throwing out noisy or unusual cardiac cycles, and by taking a running average of the signals from multiple cardiac cycles in phase with each other, for example taking a running average over 9 cardiac cycles. As described in related PCT application PCT/IL2007/001421, cited above, published as WO2008/072223, the result is a relatively low noise signal as a function of cardiac phase, which rises over a relatively short rise time from its minimum value at the diastolic point to a maximum value at the systolic point, and then falls over a longer fall time back to its minimum value at the next diastolic point. Examples of such detrended and averaged IPG and PPG signals are shown below in FIGS. 6A and 6B. The signal used for the analysis need not be a linearly amplified signal coming directly from the IPG electrodes and PPG sensors, but may be nonlinearly distorted in any manner.

An effective robust rise time interval may be defined, which may further reduce the effect of noise on the signal analysis. For example, the robust rise time interval begins when the signal is a certain fraction of its total range (maximum minus minimum) above the minimum value, for example 5% or 10% or 15% or 20% above the minimum. The robust rise time interval optionally ends when the signal first reaches a point a certain fraction of its total range below the maximum, for example 5%, 10%, 15%, 20%, 25% or 30% below the maximum. For the data analyzed below in the Examples, the robust rise time interval is defined as extended from a point 10% above the minimum to a point 20% below the maximum.

Other effective rise times are defined as ending at the point of maximum slope, or at the first local peak. With these definitions of the end of the effective rise time, the rise time interval, and other quantities which depend on it, may be less subject to being changed by noise, than if the rise time were defined as ending at the global maximum of the signal.

Characteristics of the signal in an effective rise time interval may be compared to similar characteristics of the signal in an effective fall time interval, which may optionally be defined as any part of the cardiac cycle excluding the effective rise time interval. For example, a ratio of the effective rise time interval to the effective fall time interval may be calculated, or a ratio of the signal integrated over the effective rise time interval to a ratio of the signal over the effective fall time interval. Such ratios are respectively related in a simple way to the effective rise time normalized to the whole cardiac period, and to the signal integrated over the effective rise time, normalized to the signal integrated over the whole cardiac period. The latter measure has been found to be particularly useful for estimating some standard cerebral hemodynamic parameters, as is described below in the Examples.

Another measure used in the Examples is a normalized curvature of the signal during an effective rise time interval. The curvature is defined, for example, by first fitting the signal during the rise time interval to a straight line, then fitting the signal during the rise time interval to a parabola, and taking the difference in the cardiac phase, or time, where the two fits cross a level halfway between the minimum and maximum of the signal. This difference may be normalized to the length of the rise time interval. This definition of curvature may be less sensitive to noise than simply taking the average second derivative of the signal during an effective rise time interval.

It may be useful to compare measures calculated by the same or substantially the same algorithm from two different signals, and this can serve as a measure based on both signals. (Two algorithms may be considered substantially the same if they yield similar results from a given signal, at least for most signals that are likely to occur.) For example, if the measure for each signal is an effective rise time defined in a particular way, then a measure based on two signals could be the ratio of the effective rise time for the first signal, to the effective rise time defined in the same way, or substantially the same way, for the second signal. Similarly, if the measure for each signal is the normalized signal integrated over the robust rise time described above, then the measure based on both signals could be the ratio of that normalized integral for the first signal, to the normalized integral from the second signal, defined in the same way, or substantially the same way. The two signals could be, for example, an IPG signal and a PPG signal measured on the same side of the head, or an IPG signal measured symmetrically across the head and a PPG signal measured on one side of the head, or two signals of the same modality measured on opposite sides of the head. If the measure only uses a signal measured on one side of the head, then the signal may be on the same side of the head as the suspected stroke, based on clinical data such as hemiplegia, or it may be on the opposite side of the head from the suspected stroke. It should be noted that blood circulation patterns on the side of the head opposite to a stroke are also generally affected by the stroke, because, for example, an ischemia on one side of the head may cause greater than normal blood flow on the other side of the head.

As used herein, a procedure is said to comprise comparing two signals when the procedure comprises calculating a difference between the two signals, or calculating a ratio of the two signals, or calculating any quantity that depends on how the two signals are different from each other.

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions, illustrate some embodiments of the invention in a non limiting fashion.

A clinical study was performed, using stroke patients, in which certain standard cerebral hemodynamic parameters were measured by perfusion CT, and were also estimated using various dimensionless measures based on IPG and PPG signals as functions of cardiac cycle phase. The IPG electrodes and PPG sensors were configured as shown in FIG. 3, and the IPG signals were found using up to 1 mA of current, at about 25 kHz. The signals were detrended, setting their minimum for each cardiac cycle to the same level, and in some but not all cases several consecutive cardiac cycles were averaged together, in phase, to reduce noise while retaining the shape of the signal as a function of cardiac cycle phase. A best linear fit and correlation were calculated for the dimensionless measures based on the IPG and PPG signals, and the values of the parameters measured by perfusion CT. Correlations found ranged from approximately 0.5 to 0.7, with values of the parameters generally ranging over a factor of about 2 or 3, or occasionally more, among the different patients in the sample. The best linear fits listed here could be used as a starting point for providing estimates of these cerebral hemodynamic parameters from IPG and PPG data. For example, if the normalized integral over the robust rise time is found to be exactly the same for the IPG signal and the PPG signal on the same side of the head as the stroke, then measure #2, in the list below, would be equal to 1.0, and substituting this value for the measure into the best linear fit for measure #2, Measure = -Parameter/6.9 + 1.49, would imply that the value of the parameter, in this case global CBV, is probably about 3.5. Standard units are used for the parameters: milliliters per 100 grams of tissue for CBV, milliliters per 100 grams of tissue per minute for CBF, and seconds for TTP.

1) This measure was the ratio of a measure based on the PPG signal across the head, to a measure based on the PPG signal on the side of the head opposite to the stroke. For each of these signals, the measure was a rise time interval starting at the diastolic point, and ending at the point of maximum slope. This measure was used to estimate the parameter hemispheric CBV on the stroke side. The correlation was $R^2 = 0.54$, and the best linear fit was:

$$\text{Measure} = \text{Parameter}/4.8 + 0.06$$

with the hemispheric CBV ranging from about 2 to 4.5 milliliters per 100 grams, for the bulk of the patients in the sample.

2) This measure was the ratio of a measure based on the IPG signal across the head, to a measure based on the PPG signal on the same side of the head as the stroke. For each of these signals, the measure was the normalized integral of the signal over the robust rise time interval, defined above. This measure was used to estimate the parameter global CBV. The correlation was $R^2 = 0.72$, and the best linear fit was:

$$\text{Measure} = -\text{Parameter}/6.9 + 1.49$$

with the global CBV ranging from about 2 to 4.5 milliliters per 100 grams, for the bulk of the patients in the sample.

3) This measure was the ratio of a measure based on the IPG signal across the head, to a measure based on the PPG signal on the opposite side of the head from the stroke. For each of these signals, the measure was the normalized integral of the signal over the robust rise time interval, defined above. This measure was used to estimate the parameter global CBV. The correlation was $R^2 = 0.59$, and the best linear fit was:

$$\text{Measure} = -\text{Parameter}/8.3 + 1.4$$

4) This measure was the normalized integral of the signal over the robust rise time interval, defined above, for the PPG signal on the same side of the head as the stroke. This measure was used to estimate hemispheric CBF on the same side of the head as the stroke. The correlation was $R^2 = 0.56$, and

the best linear fit was:

$$Measure = Parameter/650 + 0.12$$

with the hemispheric CBF ranging from 13 to 40 milliliters per 100 grams per minute, for the bulk of the patients in the sample.

5) This measure was the normalized integral of the signal over the robust rise time interval, defined above, for the PPG signal on the same side of the head as the stroke. This measure was used to estimate hemispheric TTP on the same side of the head as the stroke. The correlation was $R^2 = 0.56$, and the best linear fit was:

$$Measure = Parameter/420 + 0.08$$

with the hemispheric TTP ranging from 20 to 40 seconds, for the bulk of patients in the sample.

6) This measure was the normalized integral of the signal over the robust rise time interval, defined above, for the IPG signal across the head. This measure was used to estimate global TTP. The correlation was $R^2 = 0.46$, and the best linear fit was:

$$Measure = Parameter/280 + 0.04$$

with the global TTP ranging from 25 to 35 seconds, for the bulk of the patients in the sample.

7) This measure was the normalized rise time curvature of the signal, defined above, for the PPG signal on the same side of the head as the stroke. This measure was used to estimate the ratio of regional CBF on the same side of the head as the stroke, to global CBF, a quantity with a range of about a factor of 8 over the patients in the sample. The correlation was $R^2 = 0.53$, and the best linear fit was:

$$Measure = Parameter/21.6 + 0.017$$

with the ratio of regional to global CBF ranging from 0.1 to 0.8 for the bulk of the patients in the sample.

The relatively high correlations found between the measures of the IPG and PPG signals, and the cerebral hemodynamic parameters, show that it is already feasible to obtain useful estimates of cerebral hemodynamic parameters from IPG and PPG signals. In the near future, when more refined techniques for measuring IPG and PPG signals, and better measures derived from those signals, may be available, even more precise estimates of cerebral hemodynamic parameters may be possible.

FIGS. 6A and 6B show plots of IPG and PPG signals for two ischemic stroke patients who participated in the clinical study. FIG. 6A shows a plot 600 of the IPG signal measured across the head, and a plot 602 of the PPG signal measured on the same side of the head as the stroke, for a patient with unusually high global CBV, 5.3 milliliters per 100 grams of tissue, as measured by perfusion CT. The time is given in minutes, and the amplitudes of the signals are in arbitrary units. Noise has been reduced by taking a running average over 9 cardiac cycles, adding up the different cardiac cycles in phase. FIG. 6B shows a plot 604 of an IPG signal, and a plot 606 of a PPG signal, measured in the same way for a patient with unusually low global CBV, only 2.1 milliliters per 100 grams of tissue. The signals, especially the IPG signal, are visibly very different in the two patients, reflecting the large differences in their global CBV. The differences may be quantified by taking the normalized integral of the signal over a robust rise time, as described above. This quantity is 0.08 for the signal in plot 600, because the signal rises very quickly; 0.14 for the signal in plot 602; 0.21 for the signal in plot 604, which rises much more slowly than the signal in plot 600; and 0.19 for the signal in plot 606. The ratio of this quantity for the two signals provides a measure of 0.6 for the first patient, with global CBV parameter equal to 5.3, and a measure of 1.1 for the second patient, with global CBV parameter equal to 2.1. These quantities fit fairly well with the best linear fit, Measure = -Parameter/6.9 + 1.49, found for this parameter and measure in the clinical study, and one could have inferred the global CBV for these patients to fairly good approximation based on this relationship and on the IPG and PPG signals, even without making the much more expensive perfusion CT measurement.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

To the extent that section headings are used, they should not be construed as necessarily limiting.

## Claims

1. A system for evaluating an acute ischemic stroke in a patient, comprising:

   a) an electric current source;
   b) at least two sensors (108,300) adapted to be placed on the patient's head, each sensor (108,300) comprising an IPG electrode structure (304) adapted to pass current from the current source through the head to measure impedance, or comprising a PPG sensor (306) powered by the current source, or both; and

c) a controller (106) which is adapted to receive one or more waveforms of one or more signals from the sensors (108,300), adapted to process the waveforms to obtain one or more measures of cerebral hemodynamics of the patient, and is programmed with software that, when executed on the controller (106), applies a rule to match said measures to a disease indication or choice of therapy or both, for the patient;

wherein the measures comprise a measure of cerebral, blood volume (CBV), cerebral blood flow (CBF), and time to peak (TTP), and wherein applying blood flow and the rule comprises determining whether the patient suffers from ischemic stroke.

2. A system according to claim 1, wherein applying the rule comprises matching to a choice of thrombolytic therapy, or matching to a choice not to use thrombolytic therapy.

3. A system according to claim 2, wherein applying the rule comprises matching to a choice not to use thrombolytic therapy if hemispheric CBF is above an hCBF threshold.

4. A system according to claim 3, wherein the hCBF threshold is between 20 and 50 milliliters per 100 grams per minute.

5. A system according to claim 2, wherein applying the rule comprises matching to a choice not to use thrombolytic therapy if a ratio of hemispheric to global CBF is above an hCBF ratio threshold.

6. A system according to claim 5, wherein the hCBF ratio threshold is between 80% and 50%.

7. A system according to claim 2, wherein applying the rule comprises matching to a choice not to use thrombolytic therapy if hemispheric TTP is below an hTTP threshold.

8. A system according to claim 2, wherein applying the rule comprises matching to a choice not to use thrombolytic therapy if a ratio of hemispheric to global TTP is below an hTTP ratio threshold.

9. A system according to claim 8, wherein the hTTP ratio threshold is between 1.1 and 2.

10. A system according to claim 2, wherein applying the rule comprises matching to a choice not to use thrombolytic therapy if hemispheric CBV is below an hCBV threshold.

11. A system according to claim 10, wherein the hCBV threshold is between 3 and 4 milliliters per 100 grams.

12. A system according to claim 2, wherein applying the rule comprises matching to a choice not to use thrombolytic therapy if a ratio of hemispheric to global CBV is below an hCBV ratio threshold.

13. A system according to claim 12, wherein the hCBV ratio threshold is between 80% and 50%.

14. A system according to claim 4, wherein applying the rule comprises matching to a choice not to use thrombolytic therapy if regional CBV is above an rCBV threshold.

15. A system according to claim 14, wherein the rCBV threshold is between 1.5 and 2.5 milliliters per 100 grams.

16. A system according to claim 2, wherein applying the rule comprises matching to a choice not to use thrombolytic therapy if a ratio of regional to global CBV is above an rCBV ratio threshold.

17. A system according to claim 16, wherein the rCBV ratio threshold is between 30% and 75%.

18. A system according to any of claims 1-17, wherein the measures comprise an estimate of one or more of global, hemispheric and regional measures of cerebral blood flow (CBF), of cerebral blood volume (CBV), of mean transit time (MTT), and of time to peak (TTP), and mathematical functions of the foregoing parameters singly or in any combination.

19. A system according to any of claims 1-18, **characterized in that** applying the rule comprises matching at least the measure of CBV to a size of an ischemia, and matching at least the measure of one or both of CBF and TTP to an extent of a penumbra.

**Patentansprüche**

1. System zum Beurteilen eines akuten ischämischen Schlaganfalls bei einem Patienten, umfassend:

a) eine elektrische Stromquelle;
b) zumindest zwei Sensoren (108, 300), die so eingerichtet sind, dass sie auf dem Kopf des Patienten platziert werden, wobei jeder Sensor (108, 300) eine IPG-Elektrodenstruktur (304) umfasst, die so eingerichtet ist, dass sie Strom für eine Impedanzmessung von der Stromquelle durch den Kopf leitet, oder einen PPG-Sensor (306) umfasst, der von der Stromquelle mit Strom versorgt wird, oder beides; und
c) eine Steuereinheit (106), die so eingerichtet ist, dass sie eine oder mehrere Wellenformen eines oder mehrerer Signale von den Sensoren (108, 300) empfängt, die so eingerichtet ist, dass sie die Wellenformen verarbeitet, um eine oder mehrere Messwerte für zerebrale Hämodynamik des Patienten zu erhalten, und die mit Software programmiert ist, die, wenn auf der Steuereinheit (106) ausgeführt, eine Regel anwendet, um die Messwerte zu einer Krankheitsindikation oder einer Therapiewahl oder beidem für den Patienten zuzuordnen;

wobei die Messwerte einen Messwert für das zerebrale Blutvolumen (CBV), den zerebralen Blutfluss (CBF) und Time-to-Peak (TTP) umfassen, und wobei das Anwenden der Regel das Bestimmen, ob der Patienten an einem ischämischen Schlaganfall leidet, umfasst.

2. System nach Anspruch 1, wobei das Anwenden der Regel das Zuordnen zu einer Wahl für eine thrombolytische Therapie oder das Zuordnen zu einer Wahl gegen den Einsatz einer thrombolytischen Therapie umfasst.

3. System nach Anspruch 2, wobei das Anwenden der Regel das Zuordnen zu einer Wahl gegen den Einsatz einer thrombolytischen Therapie umfasst, wenn der hemisphärische CBF über einem hCBF-Schwellenwert liegt.

4. System nach Anspruch 3, wobei der hCBF-Schwellenwert zwischen 20 und 50 Milliliter pro 100 Gramm pro Minute liegt.

5. System nach Anspruch 2, wobei das Anwenden der Regel das Zuordnen zu einer Wahl gegen den Einsatz einer thrombolytischen Therapie umfasst, wenn ein Verhältnis von hemisphärischem zu globalem CBF über einem hCBF-Verhältnis-Schwellenwert liegt.

6. System nach Anspruch 5, wobei der hCBF-Verhältnis-Schwellenwert zwischen 80 % und 50 % liegt.

7. System nach Anspruch 2, wobei das Anwenden der Regel das Zuordnen zu einer Wahl gegen den Einsatz einer thrombolytischen Therapie umfasst, wenn der hemisphärische TTP unter einem hTTP-Schwellenwert liegt.

8. System nach Anspruch 2, wobei das Anwenden der Regel das Zuordnen zu einer Wahl gegen den Einsatz einer thrombolytischen Therapie umfasst, wenn ein Verhältnis von hemisphärischem zu globalem TTP unter einem hTTP-Verhältnis-Schwellenwert liegt.

9. System nach Anspruch 8, wobei der hTTP-Verhältnis-Schwellenwert zwischen 1,1 und 2 liegt.

10. System nach Anspruch 2, wobei das Anwenden der Regel das Zuordnen zu einer Wahl gegen den Einsatz einer thrombolytischen Therapie umfasst, wenn das hemisphärische CBV unter einem hCBV-Schwellenwert liegt.

11. System nach Anspruch 10, wobei der hCBV-Schwellenwert zwischen 3 und 4 Milliliter pro 100 Gramm liegt.

12. System nach Anspruch 2, wobei das Anwenden der Regel das Zuordnen zu einer Wahl gegen den Einsatz einer thrombolytischen Therapie umfasst, wenn ein Verhältnis von hemisphärischern zu globalem CBV unter einem hCBV-Verhältnis-Schwellenwert liegt.

13. System nach Anspruch 12, wobei der hCBV-Verhältnis-Schwellenwert zwischen 80 % und 50 % liegt.

14. System nach Anspruch 4, wobei das Anwenden der Regel das Zuordnen zu einer Wahl gegen den Einsatz einer thrombolytischen Therapie umfasst, wenn das regionale CBV über einem rCBV-Schwellenwert liegt.

15. System nach Anspruch 14, wobei der rCBV-Schwellenwert zwischen 1,5 und 2,5 Milliliter pro 100 Gramm liegt.

16. System nach Anspruch 2, wobei das Anwenden der Regel das Zuordnen zu einer Wahl gegen den Einsatz einer thrombolytischen Therapie umfasst, wenn ein Verhältnis von regionalem zu globalem CBV über einem rCBV-Verhältnis-Schwellenwert liegt.

17. System nach Anspruch 16, wobei der rCBV-Verhältnis-Schwellenwert zwischen 30 % und 75 % liegt.

18. System nach einem der Ansprüche 1 bis 17, wobei die Messwerte einen Schätzwert von einem oder mehreren von globalen, hemisphärischen und regionalen Messwerten für zerebralen Blutfluss (CBF), zerebrales Blutvolumen (CBV), mittlere Durchgangszeit (MTT) und Time-to-Pealc (TTP) und mathematische Funktionen der vorstehenden Parameter einzeln oder in einer beliebigen Kombination umfassen.

19. System nach einem der Ansprüche 1 bis 18, **dadurch gekennzeichnet, dass** das Anwenden der Regel das Zuordnen zumindest des CBV-Messwerts zu einer Größe einer Ischämie und das Zuordnen zumindest des Messwerts für eines oder beides von CBF und TTP zu einem Grad einer Penumbra umfasst.

**Revendications**

1. Système pour évaluer un accident vasculaire cérébral ischémique chez un patient, comprenant :

   a) une source de courant électrique ;
   b) au moins deux capteurs (108, 300) adaptés pour être placés sur la tête du patient, chaque capteur (108, 300) comprenant une structure d'électrode IPG (304) adaptée pour faire passer du courant depuis la source de courant à travers la tête pour mesurer l'impédance, ou comprenant un capteur PPG (306) alimenté par la source de courant, ou les deux ; et
   c) un dispositif de commande (106) qui est adapté pour recevoir une ou plusieurs formes d'onde d'un ou plusieurs signaux depuis les capteurs (108, 300), adapté pour traiter les formes d'onde pour obtenir une ou plusieurs mesures d'hémodynamie cérébrale du patient, et est programmé avec un logiciel qui, lorsqu'il est exécuté sur le dispositif de commande (106), applique une règle pour mettre en correspondance lesdites mesures avec une indication de maladie ou un choix de thérapie ou les deux, pour le patient ;

   les mesures comprenant une mesure de volume sanguin cérébral (VSC), de débit sanguin cérébral (DSC), et de temps de crête (TDC) et l'application de la règle comprenant la détermination du fait que le patient souffre ou non d'un accident vasculaire cérébral ischémique.

2. Système selon la revendication 1, l'application de la règle comprenant l'adoption d'un choix de thérapie thrombolytique, ou l'adoption d'un choix de ne pas utiliser une thérapie thrombolytique.

3. Système selon la revendication 2, l'application de la règle comprenant l'adoption d'un choix de ne pas utiliser une thérapie thrombolytique si le DSC hémisphérique est au-dessus d'un seuil DSCh.

4. Système selon la revendication 3, le seuil DSCh étant compris entre 20 et 50 millilitres par 100 grammes par minute.

5. Système selon la revendication 2, l'application de la règle comprenant l'adoption d'un choix de ne pas utiliser une thérapie thrombolytique si un rapport de DSC hémisphérique à global est au-dessus d'un seuil de rapport DSCh.

6. Système selon la revendication 5, le seuil de rapport DSCh étant compris entre 80 % et 50 %.

7. Système selon la revendication 2, l'application de la règle comprenant l'adoption d'un choix de ne pas utiliser une thérapie thrombolytique si TDC hémisphérique est au-dessous d'un seuil TDCh.

8. Système selon la revendication 2, l'application de la règle comprenant l'adoption d'un choix de ne pas utiliser une thérapie thrombolytique si un rapport de TDC hémisphérique à global est au-dessous d'un seuil de rapport TDCh.

9. Système selon la revendication 8, le seuil de rapport TDCh étant compris entre 1,1 et 2.

10. Système selon la revendication 2, l'application de la règle comprenant l'adoption d'un choix de ne pas utiliser une thérapie thrombolytique si VSC hémisphérique est au-dessous d'un seuil VSCh.

11. Système selon la revendication 10, le seuil VSCh étant compris entre 3 et 4 millilitres pour 100 grammes.

12. Système selon la revendication 2, l'application de la règle comprenant l'adoption d'un choix de ne pas utiliser une thérapie thrombolytique si un rapport de VSC hémisphérique à global est au-dessous d'un seuil de rapport VSCh.

13. Système selon la revendication 12, le seuil de rapport VSCh est compris entre 80 % et 50 %.

14. Système selon la revendication 4, dans lequel l'application de la règle comprend l'adoption d'un choix de ne pas utiliser une thérapie thrombolytique si VSC régional est au-dessus d'un seuil VSCr.

15. Système selon la revendication 14, le seuil VSCr étant compris entre 1,5 et 2,5 millilitres pour 100 grammes.

16. Système selon la revendication 2, l'application de la règle comprenant l'adoption d'un choix de ne pas utiliser une thérapie thrombolytique si un rapport de VSC régional à global est au-dessus d'un seuil de rapport VSCr.

17. Système selon la revendication 16, dans lequel le seuil de rapport VSCr est compris entre 30 % et 75 %.

18. Système selon l'une quelconque des revendications 1 à 17, les mesures comprenant une estimation de l'une ou plusieurs des mesures globales, hémisphériques et régionales de débit sanguin cérébral (DSC), de volume sanguin cérébral (VSC), de temps de transit moyen (TTM) et de temps de crête (TDC), et des fonctions mathématiques des paramètres ci-dessus seuls ou dans une combinaison quelconque.

**19.** Système selon l'une quelconque des revendications 1 à 18, **caractérisé en ce que** l'application de la règle comprend l'adaptation d'au moins la mesure de VSC à une taille d'une ischémie, et l'adaptation d'au moins la mesure de l'un ou les deux de DSC et TDC à l'étendue d'une pénombre.

FIG. 1

200

**202**
Evaluate clinical condition

**204**
Apply IPG electrodes and PPG sensors

**206**
Obtain signals

**208**
Process signals

**210**
Display estimated cerebral hemodynamic parameters

**220**
Initiate appropriate medical tests and treatment

**212**
Ischemic stroke? — No

Yes

**214**
Too massive to benefit from thrombolytic therapy? — Yes

No

**216**
Too small to benefit from thrombolytic therapy? — Yes / No

**218**
Begin thrombolytic therapy

FIG. 2

FIG. 3

FIG. 4

FIG. 5

FIG. 6A

EP 2 344 033 B1

EP 2 344 033 B1

FIG. 6B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 11610553 B
- US 20070287899 A
- WO 2008072223 A
- WO 03059164 A
- US 20050054939 A
- WO 2006006143 A
- WO 2006011128 A
- WO 2006134501 A
- WO 200807223 A
- WO 02071923 A, Bridger
- WO 02087410 A, Naisberg
- US 10893570 B
- IL 2005000632 W
- IL 2007001421 W

### Non-patent literature cited in the description

- **CHRISTIAN BAUMGARTNER et al.** Functional Cluster Analysis of CT Perfusion Maps: A New Tool for Diagnosis of Acute Strokes. *J. of Digital Imaging,* 2005, vol. 18, 219-226
- **ROLAND BRUENING ; AXEL KUETTNER ; THOMAS FLOHR.** Protocols for Multislice CT. Springer, 2005, 96
- **ELLEN G. HOEFFNER et al.** Cerebral Perfusion CT: Technique and Clinical Applications. *Radiology,* 2004, vol. 231, 632-644
- **HIROSHI HAGIWARA et al.** Predicting the Fate of Acute Ischemic Lesions Using Perfusion Computed Tomography. *J. Comput. Assist. Tomogr.,* 2008, vol. 32, 645-650
- **A. M. WEINDLING ; N. MURDOCH ; P. ROLFE.** Effect of electrode size on the contributions of intracranial and extracranial blood flow to the cerebral electrical impedance plethysmogram. *Med. & Biol. Eng. & Comput.,* 1982, vol. 20, 545-549
- **J. GRONLUND ; J. JALONEN ; I. VALIMAKI.** Transcephalic electrical impedance provides a means for quantifying pulsatile cerebral blood volume changes following head-up tilt. *Early Human Development,* 1997, vol. 47, 11-18
- **J. GRONLUND et al.** High Frequency Variability of Transcephalic Electrical Impedance: A New Parameter for Monitoring of Neonatal Cerebral Circulation?. *Proceedings of the Annual International Conference of the Engineering in Medicine and Biology Society,* 29 October 1992, vol. 6, 2513-2515
- **W. TRACZEWSKI et al.** The Role of Computerized Rheoencephalography in the Assessment of Normal Pressure Hydrocephalus. *J. Neurotrauma,* 2005, vol. 22, 836-843
- **G. BONMASSAR ; S. IWAKI.** The Shape of Electrical Impedance Spectrosopy (EIS) is altered in Stroke Patients. *Proceedings of the 26th Annual Conference of IEEE EMBS,* 01 September 2004
- **YU. E. MOSKALENKO et al.** Slow Rhythmic Oscillations within the Human Cranium: Phenomenology, Origin, and Informational Significance. *Human Physiology,* 2001, vol. 27, 171-178
- **A. RAGAUSKAS et al.** Implementation of non-invasive brain physiological monitoring concepts. *Medical Engineering and Physics,* 2003, vol. 25, 667-687
- **KIDWELL CS et al.** Comparison of MRI and CT for detection of acute intracerebral hemorrhage. *JAMA,* 2004, vol. 292, 1823-1830
- **HOROWITZ SH et al.** Computed tomographic-angiographic findings within the first 5 hours of cerebral infarction. *Stroke,* 1991, vol. 22, 1245-1253
- Association of outcome with early stroke treatment: Pooled analysis of ATLANTIS, ECASS, and NINDS rt-PA stroke trials. *Lancet,* vol. 363, 768-774
- **ALBERS G et al.** Antithrombotic and thrombolytic therapy for ischemic stroke: The seventh ACCP conference on antithrombotic and thrombolytic therapy. *Chest,* 2004, vol. 126, 483-512
- **KOHRMANN M.** MRI versus CT-based thrombolysis treatment within and beyond the 3 hour time window after stroke onset: a cohort study. *Lancet Neurol,* 2006, vol. 5, 661-667
- **ALBERS GW et al.** Magnetic resonance imaging profiles predict clinical response to early reperfusion: The diffusion and perfusion imaging evaluation for understanding stroke evolution (DEFUSE) study. *Ann Neurol,* 2006, vol. 60, 508-517
- **JOHNSTON SC et al.** National stroke association guidelines for the management of transient ischemic attacks. *Ann Neurol,* 2006, vol. 60, 301-313